# EUROPEAN PATENT SPECIFICATION

(11) **EP 2 498 750 B1**
(45) Date of publication and mention of the grant of the patent: **02.03.2016**
(21) Application number: 10828725.1
(22) Date of filing: 01.10.2010
(51) Int. Cl.: A61K 8/97, A61K 8/36, A61K 8/64, A61Q 1/10, A61Q 5/12

(54) **COMPOSITIONS AND METHODS FOR ENHANCING EYELASHES**
ZUSAMMENSETZUNGEN UND VERFAHREN ZUR VERSTÄRKUNG DER WIMPERN
COMPOSITIONS ET PROCÉDÉS D'AUGMENTATION DES CILS

(30) Priority: 09.11.2009 US 259419 P
(43) Date of publication of application: 19.09.2012
(73) Proprietor: Avon Products, Inc., New York, NY 10017 (US)
(72) Inventor: FELTER, Nancy, West Nyack NY 10994 (US); BUCKRIDGE, Kenneth A., North Reading MA 01864 (US); SANTHANAM, Uma, Tenafly NJ 07670 (US); GETAHOUN, Tameru, Parsippany NJ 07054 (US); HWANG, Cheng, S., New Milford NJ 07646 (US)
(74) Representative: Dr. Weitzel & Partner
(86) International application number: PCT/US2010/051063
(87) International publication number: WO 2011/056330

(56) References cited:
- WO-A2-2009/043341
- WO-A2-2009/068351
- US-A1- 2009 111 880
- US-A1- 2009 169 652
- US-A1- 2010 291 018
- AVON: 'Age Retreat Revitalizing Treatment' ADVANCE TECHNIQUES, [Online] 30 July 2009, XP055169373 Retrieved from the Internet: <URL:http://www.avon.com/1/1/12237-advance- techniques-age-retreat-revitalizing-treatme nt.html> [retrieved on 2010-11-29]

## Description

### FIELD OF INVENTION

The present invention relates generally to compositions for topical application to eyelashes which comprise creatine and low levels of a biotinylated tripeptide, as well as the use of such compositions to provide specific benefits to the eyelashes.

### BACKGROUND OF THE INVENTION

Consumers continually seek to improve the appearance of their eyelashes. Concerns include thinning or sparse eyelashes, eyelashes that are not as long as desired, or that otherwise lack a healthy, full appearance. Thus there remains a need for eye products that corrects such deficiencies.

The eyelash has two distinct structures the hair follicle, which resides in the skin; and the hair shaft or hair fiber, visible above the skin. The hair follicle is the living pan of the eyelash and contains multiple cell types such as keratinocytes and melanocytes that act to produce the hair shaft and modulate eyelash growth. Eyelash hair follicles undergo a repetitive sequence of growth and rest known as the "hair growth cycle" or "lash growth cycle" and is distinct from that of scalp hair follicles. Stern KS et al. Physiol Rev. 2001 Jan; 81(1):449-494. This growth cycle and the overall health of the lash may be modulated by internal factors, including hormones, cytokines, chemokines, and enzymes, as well as external stressors, such as UV radiation, heat, and chemical insult that can influence lash quality.

In general, eyelashes have been investigated far less than scalp hair. The lash growth cycle has been estimated to be about five to six months, with about 30-60 days in the anagen or growth phase, about 15 days in the catagen or intermediate phase, and about 3 months in the telogen or resting phase. In contrast, scalp or terminal hair has a growing phase of two to six years, an intermediate phase of several weeks, and a resting phase of several months. It appears that the eyelashes are particularly different from scalp hair with respect to a much longer resting phase relative to the growth phase.

Various approaches have attempted to enhance eyelashes using one or more bioactive ingredients implicated in lash growth cycle. Sephora CARGO LashActivator™ products, for example, uses a combination of the biotinylated tripeptide, N-biotinyl-gly-his-lys with apigenin. The tripeptide, is used at relatively high concentrations to mimic its use on scalp hair; while the apigenin is thought necessary to improve microcirculation to the hair follicles. Nonetheless, the potential vascular effects of apigenin make it unsuitable for use in some applications, and it would further be desirable to use lower amounts of the peptide-biotin complex. Moreover, many eyelash products, including CARGO LashActivaior™, have overlooked targeting one of the main constituents of the lash hair fiber, namely keratin. In particular, keratin 5 has been found localized on the outer root sheath, which is an essential part of the lash hair follicle.

Accordingly, there remains a need for eyelash enhancing cosmetic compositions that are free of unwanted ingredients, such as apigenin, use lower amounts of actives, and address lash health through additional avenues. It is therefore an object of the invention to provide such compositions and methods for improving overall appearance and health of the eyelashes. It is a further object of the invention to provide specific unique benefits to the eyelashes, traditionally overlooked in prior approaches.

The foregoing discussion is presented solely to provide a better understanding of the nature of the problems confronting the art and should not be construed in any way as an admission as to prior art nor should the citation of any reference herein be construed as an admission that such reference constitutes "prior art" to the instant application.

### SUMMARY OF THE INVENTION

In accordance with the foregoing objectives and others, it has surprisingly been found that a composition comprising creatine and low amounts of N-biotinyl-gly-his-lys is capable of synergistically increasing the number of visible eyelashes, as well as improving lash shine, volume, and length; and thus is beneficial for enhancing the appearance of eyelashes. It has further surprisingly been found that creatine stimulates the production of keratin 5, thereby increasing root sheath thickness and anchorage of eyelashes, and that these effects work synergistically with the biotinylated peptide to nourish the eyelash and improve overall eyelash health.

One aspect of the instant invention relates to compositions for topical application to the eyelashes comprising creatine, a low amount of N-biotinyl-gly-his-lys, and optionally one or more other actives as defined in the claims. The composition comprises from 1 x 10⁻⁶ to 3 x 10⁻⁴ weight % N-biotinyl-gly-his-lys. Optional actives present in some embodiments include, for example, wheat protein, a marine algae extract, tocopherol, and/or xymenynic acid. In some embodiments, the composition does not comprise xymenynic acid, or a functional amount thereof. In some embodiments, the composition does not comprise apigenin. The compositions may be provided in a cosmetically acceptable vehicle in the form of various eye products, such as preferably a serum, a mascara, of an eye liner.

Another aspect of the instant invention relates to cosmetic use of compositions described herein for improving eyelash appearance and/or health. Such improvements may comprise:
(a) improvement in lash root sheath thickness;
(b) improvement in lash anchorage;
(c) decrease in lash loss;
(d) reduction in lash breakage;
(e) increase in lash strength;
(f) improvement in lash growth rate;
(g) improvement in lash shine;
(h) improvement in the number of visible lashes;
(i) improvement in lash length; and/or
(j) improvement in lash volume.

The compositions can be applied to the eyelashes, including along the eyelash fibers and/or at the base of the lashes to contact the eyelash follicles. In some embodiments, effective amounts of creatine, N-biotyinyl-gly-his-lys, and optionally one or more other actives are provided in a cosmetically acceptable vehicle and topically applied to eyelash follicles for a time sufficient to produce the desired effect. Lash benefits may be realized immediately following application, or after a period of time of such usage. In some preferred embodiments, the inventive composition is applied to eyelashes follicles at least twice daily and/or for a period of at least 8 or at least 12 weeks.

Still another aspect of the instant invention relates to kits comprising two or more different product forms, wherein each form comprises an inventive composition. Such kits will typically include instructions, directing application of the products in a manner to provide critical doses for achieving significant improvements in eyelash health and/or appearance.

These and other aspects of the invention will be better understood by reference to the following detailed description of the invention.

### DETAILED DESCRIPTION

It has surprisingly been found that a combination of creatine and a low amount of N-biotinyl-gly-his-lys is capable of synergistically increasing lash quantity, shine, thickness, and length. It has further surprisingly been found that creatine stimulates the production of keratin 5, increasing lash root thickness and lash anchorage, and that these effects act synergistically with even low amounts of the biotinylated peptide to enhance overall eyelash health and appearance.

In view of these findings and others, a topical composition comprising creatine and a low amount of N-biotinyl-gly-lys-his is provided for use in improving eyelash appearance, and more specifically for increasing the number of visible lashes and the thickness of individual lash root sheaths. It is further contemplated that specific dosing schedules of this composition are required to produce significant and synergistic results.

### Compositions for Enhancing Eyelash Appearance

One aspect of the instant invention relates to a composition comprising a combination of actives that act synergistically to improve eyelash appearance and/or health. The combination will comprise creatine, N-biotinyl-gly-his-lys and optionally one or more other actives, including, for example, a wheat protein, an algae extract, xymenynic acid and/or tocopherol.

Creatine is a naturally-occurring amino acid derivative that is known to play a role in cellular energy metabolism. Creatine may be used in compositions described herein in any suitable form, including, e.g., creatinine and/or derivatives thereof and/or their salts. See, e.g., U.S. Pat. Appl. Pub. No. 2007/0277332. Creatine is commercially available, e.g., as Cosmocair® C100 (INCI name, hydrocreatine) or TEGO® Cosmo C100, from Evonik Industries. TEGO® Cosmo C100, for example, is sold as an additive for skin and hair formulations, and is suggested for use at a concentration of 0.5 weight % to 1.4 weight %.

N-biotinyl-gly-his-lys is a biotin complex of the tripeptide gly-his-lys, described for example in Int. Pat. Appl. Publ. WO 00/S8347, an in US 2009/169652. As used herein, "N-biotinyl-gly-his-lys" is used interchangeably with the terms "biotinylated tripeptide", "biotinylated peptide", "peptide-biotin complex", "tripeptide," and the like. It is also marketed under the trade name Procapil™, available from Sederma, Inc. Procapil™ is marketed for use in reducing scalp hair loss, in particular, scalp hair loss in men upon aging, where it is used at a concentration of about 3 weight %.

Cosmetic compositions of the instant invention will comprise an amount of each of creatine and N-biotinyl-gly-his-lys, as well as other optional actives, effective to provide a benefit to eyelashes, such as without limitation improving lash root sheath thickness and/or improving lash anchorage. The cosmetic composition comprises an amount of creatine from 0.4 to about 1.25 weight %, based on the total weight of the composition.

The cosmetic composition comprises an amount of N-biotinyl-gly-his-lys from 1 x 10⁻⁶ weight % to 3 x 10⁻⁴ weight %, or about 2 x 10⁻⁶ weight % to about 2.5 x 10⁻⁴ weight %, or about 1 x 10⁻⁵ weight % to about 2.5 x 10⁻⁴ weight %, or about 2 x 10⁻⁵ weight % to about 2 x 10⁴ weight %, based on the total weight of the composition. In some preferred embodiments, the cosmetic composition comprises at least about 1 x 10⁻⁵ weight %, or at least about 2 x 10⁻⁵ weight %, or at least 1 x 10⁻⁴ weight % N-blotinyl-gly-his-lys. The above amounts refer to an "active amount" of the creatine and biotinylated tripeptide. The term "active amount" refers to the amount of creatine, tripeptide, or other optional active, absent diluent, solvent, carrier, filler or the like. The amounts referred to with respect to other optional actives herein also refer to an active amount of the respective active.

Moreover, in certain preferred embodiments, the creatine and tripeptide components are present in the cosmetic composition in effective proportions relative to one another. Effective proportion refers to the effective amount of a given component relative to the amounts of one or more other component(s) in combination with it in the cosmetic composition. The terms "effective amount" and "amount effective", discussed above, include the concept of "effective proportion." In some particularly preferred embodiments, for example, the cosmetic composition comprises an amount of creatine of about 0.5 weight % and an amount of N-biotinyl-gly-his-lys of about 2 x 10⁻⁵ based on the total weight of the composition, or an amount of creatine of about 1 weight % and an amount of N-biotinyl-gly-his-lys of about 2 x 10⁻⁴ based on the total weight of the composition..

Surprisingly, it has been found that the combination of the biotinylated peptide with creatine allows for much lower effective amounts of the tripeptide compared to prior approaches. Such effective amounts are also surprisingly lower than that suggested by the literature for N-biotyinyl-gly-his-lys. Further, it has been surprisingly found that the combination allows for lower effective amounts of the creatine component as well, compared to prior usage.

Compositions of the instant invention may further comprise one or more optional actives, in combination with the creatine and biotinylated tripeptide components. Additional optional actives will also generally be present in effective amounts and/or in effective proportions relative to the creatine, tripeptide, and/or other components of the cosmetic compositions. Further, the effective amounts of one or more of the optional actives may be lowered, again due to the synergistic effects of the active(s) with the creatine and biotinylated peptide components.

In some embodiments, the cosmetic composition further comprises wheat proteins. Wheat protein(s) refer to one or more polypeptides extracted from wheat, and can include mixtures of wheat polypeptides and wheat oligosaccharides. In certain preferred embodiments, the wheat protein component includes a protein/starch mixture known as "Cropeptide W", INCI name "hydrolyzed wheat protein (and) hydrolyzed wheat starch", which is commercially available, e.g., from Croda, Inc., Columbus, NJ. Cropeptide W comprises a hydrolyzed wheat protein and at least partially hydrolyzed wheat oligosaccharides. The oligosaccharides are solubilized in the Cropeptide W mixture. Wheat proteins used in some embodiments, of the instant invention may comprise Cropeptide W or other polypeptide/oligosaccharide mixture, having a substantially similar composition. In some preferred embodiments, the cosmetic composition of the instant invention comprises an amount of wheat protein from about 0.0025 weight % to about 1 weight % based on the total weight of the composition: preferably from about 0.025 weight % to about 0.75 weight % based on the total weight of the composition; and more preferably from about 0.125 weight % to about 0.625 weight %, or about 0.25 weight % to about 0.5 weight %, based on the total weight of the composition.

In some embodiments, the cosmetic composition further comprises an algae extract. An algae extract, as used herein, refers to an extract of marine algae. Preferred marine algae include, for example, *Pelvetia canaliculata* and/or *Laminara digitata.* While similar marine algae extracts have been used in other cosmetic applications, such as in the Sephora lash product discussed above, they are usually used at higher relative concentrations. Accordingly, it has surprisingly been found that the combination of the tripeptide with creatine allows for much lower effective amounts of the algae extract component compared to levels at which it is conventionally used. For example, whereas a suggested usage for the algae extract is commonly about 0.35 weight %, the instant inventive combinations use as little as about 0.0015 weight %, based on the total weight of the composition. In certain preferred embodiments, the cosmetic composition of the instant invention comprises an amount of algae extract from about 1 x 10⁻⁴ weight % to about 0.3 weight % based on the total weight of the composition; preferably from about 1 x 10⁻³ weight % to about 0.2 weight %; and more preferably from about 0.01 weight % to about 0.1 weight %, or about 0.0014 to about 0.007 weight %, based on the total weight of the composition.

In some embodiments, the cosmetic composition further comprises xymenynic acid. xymenynic acid, also known as santalbic acid, is a conjugated, unsaturated fatty acid, identified in oil extracts of seeds and roots of various *Ximenia* and *Olax* species. It is an 11 - octadecen-9-ynoic acid, whose main structural feature is the presence of both a triple bold and a double bond. xymenynic acid is used in traditional pharmacopoeias, e.g., by the Australian aborigenes, and has been shown to increase blood supply to the skin when topically applied thereto. Xymenynic acid may be used in compositions described herein in any suitable form, including, e.g., its acid form or in its semi-synthetic ester derivative, xymenynic acid methyl ester. In certain preferred embodiments, the cosmetic composition comprises an amount of xymenynic acid from about 0.001 weight % to about 1 weight % based on the total weight of the compositions; preferably from about 0.05 weight % to about 0.5 weight %; and more preferably from about 0.01 weight % to about 0.1 weight %, based on the total weight of the composition. In some preferred embodiments, the cosmetic composition does not comprise xymenynic acid or does not comprise an effective amount thereof to junction in increasing topical brood supply. In still some other preferred embodiments, the cosmetic composition comprises an effective amount of xymenynic acid to function in increasing topical blood supply, such as at least about 0.0075 weight %, more preferably at least 0.01, or at least about 0.05 weight %.

In some embodiments, the cosmetic composition further comprises tocopherol. Tocopherol, or vitamin E, is known to act as an antioxidant, scavenging free radicals that can damage skin. The vitamin may be used in its active form, namely α-tocopherol, and/or as one or more of its more stable derivatives, such as its ester, tocopherol acetate and/or the form tocopheryl linoleate. Tocopherol and its derivatives are widely commercially available, e.g., tocopheryl linoleate is available from Barnet Products, Corp, NJ. In certain preferred embodiments, the cosmetic composition comprises an amount of tocopherol acetate from about 0.0001 weight % to about 1 weight % based on the total weight of the composition; preferably from about 0.0005 weight % to about 0.1 weight % based on the total weight of the composition; and more preferably from about 0.001 weight % to about 0.05 weight %, or about 0.02 weight %, based on the total weight of the composition.

Determination of additional effective amounts and/or effective proportions for the above components is within the capabilities of those skilled in the art, in light of the teachings provided herein. For example, the amounts disclosed herein, particularly amounts provided in compositional embodiments, provide representative examples. A person of ordinary skill following the teaching provided herein and using techniques known in the art also can determine other effective amounts and/or effective proportions of the components used together to impart synergistic improvements in lash appearance and/or health. The amounts disclosed, along with the teachings presented herein, provide guidance to enable one of ordinary skill in the art to select those and other effective amounts and/or proportions of the corresponding components.

In some embodiments, the cosmetic composition does not comprise apigenin. Apigenin is a citrus-derived flavonoid believed to promote microcirculation when topically applied, and has been thought necessary for optimal lash growth. Surprisingly, however, cosmetic compositions of the instant invention provide synergistic improvements in lash health and appearance in the absence of apigenin, or in the absence of an effective amount thereof. In some embodiments, the cosmetic composition is free or substantially free of apigenin meaning that no apigenin is intentionally added to the composition, although there may be trace amounts present by virtue of intrinsic presence of apigenin in other materials of the composition.

Another aspect of the invention relates to methods for making cosmetic compositions described herein. The creatine/low-tripeptide combination of the instant invention can be prepared in any conventional manner, preferably by mixing and blending the components in any order. In some preferred embodiments, the components are directly mixed into other cosmetic formulations for topical application to the eyes or eyelashes, e.g., to impart improved eyelash enhancing properties thereto. In some such embodiments, adding the creatine, N-biotinyl-gly-his-lys, and one or more optional actives to traditional cosmetic formulations has the effect of increasing pH and/or decreasing viscosity. As those of skill in the art in recognize, the pH and/or viscosity can be returned to suitable levels for cosmetic use by adding selected pH and/or viscosity modifiers.

The compositions of the instant invention find use in cosmetic formulations for topical application to the eyelash and/or eyelash follicle that can improve overall eyelash health and appearance, as described in more detail below.

### Cosmetic Use of Compositions for Enhancing Eyelash Appearance

Another aspect of the instant invention relates to cosmetic use of compositions comprising creatine and low levels of N-biotinyl-gly-his-lys. The cosmetic compositions surprisingly act synergistically to increase number of visible lashes, as well as the volume, length, and shine thereof, and accordingly find use in eye products for improving eyelash appearance and/or health.

"Improving eyelash appearance and/or health" and related expressions refer to providing at least one benefit to eyelashes, whether the benefit occurs immediately following application of a composition described herein, or after a period of time of such usage. In some embodiments, a method for providing at least one benefit to eyelashes is provided, where the method comprises topically applying to eyelashes at least one composition described herein in a cosmetically acceptable vehicle. "Applying to eyelashes" refers to applying the composition to any part of the eyelash, including the eyelash follicles, e.g., at the base of the eyelash, and/or along the length of the lash fiber above the skin. In certain preferred embodiments, the composition is applied directly to the base of the upper and/or lower eyelashes, where it may contact the eyelash follicles. For example, using a serum or eye liner, a thin line can be drawn along the base of the upper and/or lower lashes and allowed to dry.

The composition will comprise an effective amount of creatine, N-biotinyl-gly-his-lys and optionally one or more other actives, as described herein. An "amount effective" or an "effective amount" to provide a particular benefit to the eyelashes refers to an active amount of creatine in combination with an active amount of N-biotinyl-gly-his-lys sufficient to provide a clinically measurable improvement in the particular manifestation of the eyelash when applied for a sufficient time. Such benefits include without limitation, the following:
(a) improvement in lash root sheath thickness;
(b) improvement in lash anchorage;
(c) decrease in lash loss;
(d) reduction in lash breakage;
(e) increase in lash strength;
(f) improvement in lash growth rate;
(g) improvement in lash shine;
(h) improvement in the number of visible lashes;
(i) improvement in lash length; and
(j) improvement in lash volume.

The compositions of the invention can be applied to eyelashes in need of treatment, such as eyelashes which suffer from a deficiency or loss in any of the foregoing attributes, or which would otherwise benefit from the composition's eyelash enhancing effects, e.g., as described herein. For example, the creatine/low-N-biotinyl-gly-his-lys combination can be provided in a cosmetically acceptable vehicle, topically applied to the base and/or length of the eyelash, and allowed to remain on the area in an amount effective to improve the aesthetic appearance and/or health of the eyelash.

It is contemplated that benefits occur in two stages, temporally. Benefits may occur instantly, meaning that there is a visible enhancement in the lashes immediately following application of a composition described herein. Typically, for example, application of a mascara product comprising an inventive composition will show immediate or instant improvements in lash appearance. See, e.g., Example 1 below providing an example of a mascara formulation, according to the invention. Instant lash benefits can comprise, e.g., improvement in visible length and/or volume (thickness) of the lashes. See Example 3 below, Table L

Lash benefits also comprise long-term benefits, meaning benefits that develop over a period of time following usage, preferably repeated usage, of a composition described herein. Such long-term benefits can result in an improvement in eyelash appearance even in the absence of eye makeup, such as even in the absence of a mascara. Typically, for example, application of a serum product comprising an inventive composition in show long-term improvements in lash appearance. See, e.g., Example 2 below providing an example of a serum formulation, according to the invention. Long-term lash benefits can comprise, e.g., improvement in lash root sheath thickness; improvement in lash anchorage; decrease in lash loss; reduction in lash breakage; increase in lash strength; improvement in lash growth rate; improvement in lash length; improvement in lash volume; improvement in lash shine; and/or improvement in the number of visible lashes. See Example 3 below, Table 2.

An improvement in root sheath thickness refers to any increase in the individual or average thickness (diameter) of a lash within a lash follicle for a particular individual. The increase in root sheath thickness in generally translate to an increase in lash thickness, as the lash continues to grow out of the follicle and above the skin. Root sheath thickness can be measured by any means known in the art and/or described herein. In some embodiments, use of a composition described herein increases lash root sheath thickness by at least about 20%, at least about 50%, at least about 80%, at least about 100%, at least about 130%, or at least about 150%, compared to root sheath thickness for that particular root sheath for that particular individual in the absence of using the composition, In some preferred embodiments, the improvement in lash root sheath thickness further translates to an improvement in lash anchorage, as discussed below.

"Lash anchorage" as used herein refers to the stability with which a lash is secured in its follicle and/or likely to remain attached to the eyelid in the face of mechanical, chemical, physiological, and/or other stresses. "Lash anchorage" is used interchangeably herein with the term "lash rootedness" and is generally inversely related to the rate of lash loss. Lashes generally grow until reaching their maximal length and then stop growing and fall off. An increase in lash rootedness can act to delay, hinder, or prevent this falling off, decreasing in the overall rate of lash loss. An improvement in lash anchorage/rootedness, or a decrease in lash loss, then refers to any increase in the stability with which the lash remains secure in its follicle and/or any increase in the likelihood that it will remain attached to the eyelid in the face one or more stresses, rather than be lost. Lash anchorage and/or lash loss can be measured by any means known in the art and/or described herein. Lash anchorage can vary based on a number of factors, such as ethnicity, age, overall health, position of the eyelash, and the like. For example, upper eyelashes generally appear to be embedded more deeply than lower lashes. Lash anchorage also varies with the stage of the lash growth cycle, e.g., being strongest during the anagen (growth) phase and weakest approaching the telogen (resting) phase.

Without wishing to be bound by theory, it is believed that the creatine and tripeptide components of the inventive compositions act synergistically to bring about the effects of improved root sheath thickness, improved lash anchorage, and/or decreased lash loss. As noted above, the main constituents of the lash fiber are keratins. For example, it is known that a deficiency in keratin production can lead to brittle hair. In particular, keratin 5 has been shown to be localized on the outer root sheath, which is an essential part of lash follicle. Further, it is known that extracellular matrix components (EMC) are important for the repeated re-organization of the lower follicle structure during the lash growth cycle. In particular, collagen IV and fibronectin have shown to be major constituents of the basement membrane of lash follicles. Without wishing to be bound by theory, it is believed that creatine acts to increase keratin production, particularly keratin 5 production, thereby increasing lash root sheath thickness and acting to better anchor the lash in the eyelid. See Example 4 below. At the same time, it is believed that N-biotinyl-gly-his-lys acts to increase production in fibroblasts of fibronectin and collagen IV, as well as elastin, further promoting healthy lash follicles and synergistically improving lash anchorage. See Example 5 below. The combined effects of increasing keratin 5, fibronectin, collagen IV, and elastin at the lash follicle surprisingly produces a synergistic improvement in lash root sheath thickness and/or root anchorage, and a consequent synergistic decrease in lash loss.

Furthermore, N-biotinyl-gly-his-lys may also act to preserve extracellular matrix components from degradation. The degradation of extracellular matrix components is largely brought about by matrix metalloproteinases (MMPs). MMPs are a family of zinc metallo-peptidases secreted by cells that degrade extracellular matrix components and are known to largely control tissue remodeling in and around the eyelash follicle. Without wishing to be bound by theory, it is believed that N-biotinyl-gly-his-lys can act to decrease MMP-3 activity, thereby protecting extracellular matrix components of eyelash follicles from degradation. See Example 6 below. This protective action of the tripeptide component, along with its EMC-promoting actions, may further work synergistically with creatine's keratin 5-promoting activity to produce synergistic benefits in lash appearance and/or health.

Accordingly, in some particularly preferred embodiments, a composition for topical application to eyelash follicles is provided that comprises an amount of creatine sufficient to increase keratin 5 production; in combination with an amount of N-biotinyl-gly-his-lys sufficient to decrease MMP-3 activity and/or to increase fibronectin, collagen IV, and/or elastin production. In some preferred embodiments, production of keratin 5, collagen IV, fibronectin and/or elastin increases by at least about 20%, at least about 50%, at least about 80%, at least about 100%, at least about 130%, or at least about 150%, compared to production of the protein in the absence of the inventive composition. In some preferred embodiments, MMP-3 activity is decreased by at least about 20%, at least about 30%, at least about 50%, or at least about 80%, compared to activity of the enzyme in the absence of the inventive composition. Further, as noted above, the amount of the tripeptide needed may be reduced, significantly, substantially, and/or exponentially, due to the presence and complimentary action of the creatine component. The amount of creatine needed may also be reduced, due to the complimentary and synergistic action of the tripeptide component.

In certain particularly preferred embodiments, the increase in lash root sheath thickness and/or lash anchorage further leads to one or more additional lash benefits, including, e.g., a reduction in lash breakage; increase in lash strength; improvement in lash growth rate; improvement in lash length; improvement in lash volume; improvement in lash shine; and/or improvement in the number of visible lashes. Moreover, these benefits, along with the root sheath and anchorage benefits, may be further enhanced, e.g., synergistically enhanced, with the addition of one or more optional actives to the inventive compositions, as described in more detail below.

A reduction in breakage refers to any decrease in the frequency with which part of an eyelash fiber falls off, although its root remains secure in the follicle. Such a reduction in breakage is related to an increase in lash strength, as the stronger the tensile strength of a lash follicle, the less likely is it to break. Tensile strength of lashes can be measured by techniques known in the art and/or described herein. Without wishing to be bound by theory, these benefits may result from the synergistic improvement in lash thickness due to the increased production of keratin 5, collagen IV, fibronectin and/or elastin, and/or the decreased degradation of EMCs, as described above.

An improvement in lash growth rate refers to any increase in the individual or average rate at which the visible hair fiber of the lash extends above the hair follicle. In one study, the growth rate of lashes was found to be about 140µm/day, such that it would take about 2 months for an eyelash to reach its typical maximum length. Lash growth rate can be measured by techniques known in the art and/or described herein. In some embodiments, use of a composition described herein increases lash growth rate by at least about 10%, at least about 30%, at least about 50%, at least about 80%, or at least about 100%, compared to the growth rate of the lash for that particular individual in the absence of the composition.

in certain preferred embodiments, lashes show an improvement in shine and/or in the number of lashes visible, particularly following use of a composition described herein for over a period of time. "lash shine" refers to the amount of light reflected from the surface of a lash, and has the effect of producing a healthier appearance. An improvement in lash shine refers to any individual or average increase in the amount of light reflected from the surface of a bare lash, that is, a lash without any mascara or other eye product. Lash shine can be measured by techniques known in the art and/or described herein, e.g., in Example 3 below. In some embodiments, use of a composition described herein increases lash shine by at least about 10%, at least about 15%, at least about 20%, at least about 25%, or at least about 30%, compared to lash shine for that particular individual prior to using the composition. See Example 3, below Table 2. Typically results are seen following at least about 5 weeks, at least abut about 6 weeks, at least about 8 weeks, at least about 12 weeks, or more, of at least twice daily application to eyelash follicles, as described in more detail below.

An improvement in the number of visible lashes refers to any increase in the quantity of individual hair fibers that can be seen by the naked eye protruding above the lash follicle at any given point in time for a given eyelid of an individual. This improvement represent a unique benefit, traditionally not seen with other eyelash treatments. The terms "number of visible lashes", "visible number of lashes", "lash quantify", and "visible lash quantity" are used interchangeably herein. The maximum possible number of visible lashes on an eyelid corresponds with the total number of lash hair follicles on the eyelid. On average, the total number of lash hair follicles on the upper eyelid is about 150 to about 200, whereas lower lids appear to have fewer follicles. The greater the number of total follicles bearing visible lash fibers at any given time, the greater the number of visible lashes. Visible lash quantity can be measured by techniques known in the art and/or described herein, e.g., in Example 3 below. In some embodiments, use of a composition described herein increases the visible number of lashes by at least about 10%, at least about 20%, at least about 30%, at least about 50%, or at least about 70%, compared to the number visible lashes on a particular eyelid of an individual prior to using the composition. See Example 3 below. Table 2. In some embodiments, the improvement is seen for lashes on the upper eyelid, that is the "top lashes." In some embodiments, the improvement is seen for lashes on the lower eyelid, that is the "bottom lashes." In some preferred embodiments, the improvement is seen for both top and bottom lashes. Typically results are seen following at least about 5 weeks, at least abut about 6 weeks, at least about 8 weeks, at least about 12 weeks, or more, of at least twice daily application to eyelash follicles, as described in more detail below,

Without wishing to be bound by theory, it is believed that the combined actions of creatine and N-biotinyl-gly-his-lys produce a synergistic improvement in lash shine and/or visible quantity. Specifically, the combined effects of increasing keratin 5, as well as fibronectin, collagen IV, and elastin at the lash follicle produces a synergistic improvement in lash root sheath thickness, root anchorage, and a consequent synergistic decrease in lash loss; as well as a synergistic improvement in lash strength, and a consequent synergistic decrease in lash breakage, with a net improvement in the overall health of the lashes. Such health benefits surprisingly appear as synergistically increased lash shine and/or synergistically increased visible lash quantity, in certain particularly preferred embodiments.

In still further particularly preferred embodiments, use of a composition described herein results in improved lash length and/or lash volume. Lash length and/or volume may improve either immediately after applying the composition, e.g., immediately after applying a mascara product to lash fibers; and/or following use of the composition for a period of time, e.g., after repeatedly applying a serum product to lash follicles. "Lash length" is used interchangeably herein with the term "visible lash length" and "lash volume" is used interchangeably herein with the terms "visible lash volume', "lash thickness", and "visible lash thickness."

An improvement in lash length refers to any increase in the individual or average length of the visible lash fiber above the lash follicle on a given portion of the eyelid for an individual at a particular point in time. The average length of eyelashes on the upper eyelid, for example, is about 7.0 to about 7.2 mm. Lash length varies based on a number of factors, including, e.g., ethnicity, overall health, position on the eyelid, and the like. Longest lashes usually occur in the middle portion of Caucasian eyelids, for example, with shorter lashes on either end; whereas on Asian eyelids, longest lashes usually occur more toward the outer portion of the eyes. Lash length may be measured by techniques known in the art and/or described herein, e.g., as in Example 3 below. In some embodiments, use of a composition described herein increases lash length by at least about 20%, at least about 40%, at least about 50%, at least about 60%, at least about 80%, or at least about 100% compared to the length of the lash at a given portion of the eye for a particular individual in the absence of the composition. See Example 3 below, Table 1. In some embodiments, the improvement is seen for lashes on the upper eyelid, that is the "top lashes." In some embodiments, the improvement is seen for lashes on the lower eyelid, that is, the "bottom lashes." In some preferred embodiments, the improvement is seen for both top and bottom lashes.

An improvement in lash volume refers to any increase in the individual or average thickness (width or diameter) of the visible lash fiber on a given eyelid for an individual at a particular point in time. Like lash length, lash volume varies based on a number of factors, including, e.g., ethnicity, overall health, position on the eyelid, and the like. For example, upper eyelashes generally are thicker than lower lashes; and average eyelash thickness for Asian eyes (about 71 µm) tends to be greater than that for Caucasian eyes (about 61 µm). Lash volume may be measured by techniques known in the art and/or described herein, e.g.. as in Example 3 below. In some embodiments, use of a composition described herein increases lash length by at least about 30%, at least about 50%, at least about. 80%, at least about 100%, at least about 130%, or at least about 150% compared to the volume of the lash at a given portion of the eye for a particular individual in the absence of the composition. See Example 3 below, Table 1. In some embodiments, the improvement is seen for lashes on the upper eyelid, that is the "top lashes." In some embodiments, the improvement is seen for lashes on the lower eyelid, that is the "bottom lashes." In some preferred embodiments, the improvement is seen for both top and bottom lashes.

In certain preferred embodiments, the cosmetic compositions described herein further comprise one or more additional optional actives that act to further enhance eyelash appearance and/or health. In certain particularly preferred embodiments, the additional active(s) acts synergistically with the creatine and tripeptide components to bring out such additional lash benefits, as described in more detail below.

In some embodiments, the cosmetic composition further comprises wheat protein, such as described above. It is believed that the wheat protein can penetrate lash hair fibers and act as a hydroscopic moisture-balancing agent, both inside the fiber and on the hair surface. Without wishing to be bound by theory, it is further believed that the penetrating and hydroscopic actions of the wheat protein component may act to thicken the leashes and make them more resistant to mechanical damage. Greater thickness can further improve, preferably synergistically improve, root sheath thickness and/or rootedness, as well as further decreasing lash loss. Greater resistance to mechanical damage can further increase, preferably synergistically increase, lash strength, as well as further decreasing lash breakage and/or further improving lash growth rate. As detailed above, such effects can also translate to further improvement, preferably synergistic improvement, in lash length, lash volume, lash shine, and/or number of visible lashes.

In some embodiments, the cosmetic composition further comprises an algae extract, such as described above. It is believed that the algae extract can act further to protect extracellular matrix components of lash follicles. As noted above, N-biotinyl-gly-his-lys is believed to inhibit MMP-3, a member of the MMP family of zinc metallo-peptidases that degrade extracellular matrix components and largely control tissue remodeling in and around the eyelash follicles. Two additional MMPs that play key roles with respect to the lash follicle include MMP-2 and MMP-9. Without wishing to be bound by theory, it is believed that the algae extract can act to decrease MMP-2 and MM-9 activity, as well as MMP-1 and MMP-3 activity, thereby further protecting extracellular matrix components of eyelash follicles from degradation. See Example 7 below.

Accordingly, in some preferred embodiments, the composition comprises an amount of algae extract sufficient to decrease MMP-1, -2, -3, and/or -9 activity. In some preferred embodiments, MMP-1, -2, -3, and/or -9 activity is decreased by at least about 20%, at least about 50%, at least about 60%, at least about 80%, or at least about 90% compared to enzymatic activity in the absence of the inventive pomposition. It is believed that this action combines synergistically with the action of the creation component, in increasing keratin 5 production; and/or with the action of the tripeptide component, in increasing production of several extracellular matrix components and also decreasing MMP-3 activity. These combined actions may further improved, preferably synergistically improve, root sheath thickness and/or rootedness, as well as further decreasing lash loss. They may also further increase, preferably synergistically increase, lash strength, as well as further decreasing lash breakage and/or further improving lash growth rate. As detailed above, such effects can also translate to further improvement, preferably synergistic improvement, in lash length, lash volume, lash shrine, and/or number of visible lashes.

Additionally, it is believed that the algae extract may also act to maintain healthy structure and function of lash proteins by stimulating "heat shock proteins" (HSPs). Heat shock proteins (HSPs), also know as "stress proteins", are a family of highly conserved proteins found in all organisms. HSPs are induced by a wide variety of stresses, such as increased temperature, oxygen deprivation, pH changes, chemical insult, UV radiation, and the like. These stresses modify the folding structure of proteins. Improperly folded proteins lead to loss of function and potentially cell death. HSPs bind to proteins during stress to help maintain and/or restore protein structure and function. It is known that during the lash growth cycle, levels of HSPs increase during the remodeling of the hair follicle but decrease during the regression of keratinocytes in the hair bulb, and that, in particular, the isoforms HSP27 and HSP70 are found within the hair follicle. Without wishing to be bound by theory, it is believed that the algae extract increases activity of HSP27 and HSP70 at the gene level, thereby further protecting lash proteins, especially during periods of stress. See Example 8 below. Eyelash stresses include, for example, stresses from curling the eyelashes, heat, and/or removing eye makeup.

Accordingly, in some preferred embodiments, the composition comprises an amount of algae extract sufficient to increase HSP27 and/or HSP70 activity. In some preferred embodiments, HSP27 and/or HSP70 activity is increased by at least about 20%, at least about 50%, at least about 80%, at least about 100%, or at least about 120% compared to enzymatic activity in the absence of the inventive composition. It is believed that this action further combines synergistically with the actions of the creation and tripeptide components. These combined actions may further improve, preferably synergistically improve, root sheath thickness and/or rootedness, as well as further decreasing lash loss. They may also further increase, preferably synergistically increase, lash strength, as well as further decreasing lash breakage and/or further improving lash growth rate. As detailed above, such effects can also translate to further improvement, preferably synergistic improvement, in lash length, lash volume, lash shine, and/or number of visible lashes.

In some embodiments, the cosmetic composition further comprises tocopherol, such as described above. It is believed that tocopherol can act to further nourish eyelash health, for example, by protecting eyelashes from free radical damage. Reactive oxygen species generated, e.g., by UV and inflammation, have been shown to cause DNA and protein damage and cell death. It is believed that such damage can also negatively affect the lash growth cycle, lash health, or even lash pigmentation. Tocopherol has been shown to act as an antioxidant, scavenging free radicals and reducing UV-induced damage in keratinocytes and melanocytes. Werninghaus K. et al. Photodermatol Photoimmunol Photomed. 1991 Dec; 8(6): 236-42. Later *in vitro* studies demonstrated tocopherol's role in preventing UVB-induced pyrimidine dimer formation in keratinocytes; reducing the frequency of UV-induced apopiosis in melanocytes; and decreasing UV-induced IL-8 production, which is associated with activation of the oxidative stress pathway. Malka H. et al. Biomed Pharmacother. 2006 Jun: 60(5): 233-7; Larson P. et al. Br J Dermatol. 2006 Aug; 155(2): 292-300; and Wu S. et al. Mol. Immunol. 2008 Apr; 45(8): 2288-96. Another measured of free radical protection is the lipid peroxidation in a cell, where the higher the lipid peroxidation, the better the protection offered. Without wishing to be bound by theory, it is believed that tocopherol can act to increase lipid peroxidation in keratinocytes, thereby protecting the eyelash and surrounding follicle cells from free radical damage and further contributing to overall lash health. See Example 9 below.

Accordingly, in some preferred embodiments, the composition comprises an amount of tocopherol (e.g., as tocopherol acetate) sufficient to increase lipid peroxidation. In some preferred embodiments, lipid peroxidation is increased by at least about 20%, at least about 50%, at least about 80%, at least about 100%, or at least about 120% compared to lipid peroxidation in the absence of the inventive composition. It is believed that this action further combines synergistically with the activities of the creation and tripeptide components. These combined actions may further improve, preferably synergistically improve, root sheath thickness and/or rootedness, as well as further decreasing lash loss. They may also further increase, preferably synergistically increase, lash strength, as well as further decreasing lash breakage and/or further improving lash growth rate. As detailed above, such effects can also translate to further improvement, preferably synergistic improvement, in lash length, lash volume, lash shine, and/or number of visible lashes.

The invention provides methods for improving eyelash appearance and/or health by topically applying a composition comprising creatine and a low amount of N-biotinyl-gly-his-lys to eyelash fibers and/or follicles for a period of time sufficient to produce one or more of the benefits described herein. As noted above, benefits may occur instantly or over the long-term. Typically, instant benefits are realized using a mascara product for topical application to the eyelash fibers. For example, in some embodiments, a mascara comprising a composition described herein can be applied to the eyelashes. Preferably, the mascara is applied along the length of the eyelash fiber, including touching the lash base, to contact the lash follicles. Mascara can be applied to top and/or bottom lashes using, e.g., at least about 5, at least about 10, at least about 15, or a least about 20 strokes of a mascara wand, wherein the wand is preferably dipped into the mascara product between top and bottom lash applications. The initial coat can be allowed to dry, followed by application of a second coat or additional coats, e.g., in the same or similar manner as the first coat was applied. Mascara may also be "touched up" as needed during wear. See Example 3 below. In some particularly preferred embodiments, mascara application is used in conjunction with application of a serum, as described below.

Typically, long-term benefits are realized using a serum product for topical application to the eyelash follicles. For example, in some embodiments, a serum comprising a composition described herein can be applied to the base of the upper and/or lower lash line. The serum is typically clear or colorless and can be applied as a thin line along the base of the lash line, and allowed to dry, e.g., before applying mascara. Application can be repeated one, two, three or more times daily, for as long as is necessary to achieve desired results, such as the lash nourishing and/or volumizing effects described herein Preferably, the serum is applied at least twice daily, e.g., once in the morning, such as before applying mascara; and once in the evening, such as after removing mascara at night. In even more preferred embodiments, the mascara used daily also comprises a composition described herein, further increasing the daily dose. Notably, it has been surprisingly discovered that there is a criticality of dosage with respect to usage of some embodiments of the instant invention. For example, once daily application of a certain compositional embodiment did not produce significant improvement in shine and/or visible number of lashes, whereas significant improvements were observed with at least twice daily application thereof. See Example 3 below, Table 2. Such a regimen may be continued, for example, for at least about one week, at least about 2 weeks, at least about 4 weeks, at least about 5 weeks, at least about 6 weeks, at least about 8 weeks, at least about 12 weeks, or more. In some particularly preferred embodiments, the regimen involves application of a serum comprising an inventive composition twice daily, as well as application of a mascara comprising an inventive composition once daily, continued for at least about 12 weeks. See again Example 3 below.

The compositions of the invention can be applied directly to a keratin fiber, including eyelashes and eyebrows. In one embodiment, a method is provided for promoting retention of keratin fibers comprising the eyebrows, the method comprising applying an effective amount of the inventive compositions directly to the eyebrows.

In some embodiments, the use or application of the inventive compositions avoids application to the scalp or hair of the scalp.

Based on the teachings provided herein, one of skill in the art will recognize other cosmetic and/or personal care applications for the compositions described herein, and such applications are also contemplated as within the scope of the instant invention. For example, compositions described herein may also find use in eye creams or face creams for day or night use that will be topically applied to areas including the eyelids and eyelash follicles. The cosmetic compositions described herein generally find use in various eye products, such as serum, mascara, or eyeliner, formulated for topical application to the lashes and lash follicles, e.g., with a cosmetically acceptable vehicle, as described in more detail below.

### Cosmetic Formulations of Compositions for Enhancing Eyelash Appearance

The compositions described herein can be formulated as a variety of eye products for topical application to the lashes. The composition may be formulated in a variety of product forms suitable for application to the lashes, such as, for example, a lotion, cream, serum, spray, aerosol, cake, ointment, essence, gel, paste, patch, pad, eyelash strip, pencil, pomade, solution, towelette, mask, stick, foam, elixir, mousse, powder, foaming cleanser, concentrate, or any other liquid, semi-solid, or solid form. Preferably the composition is formulated as a serum, mascara or eye liner, e.g. for topical application to the eyelash including the base of the eyelash. As noted above, an exemplary formulation for a mascara is provided below in Example 1, which finds use as a volumizing mascara. An exemplary formulation for a serum is provided in Example 2 below, which finds use as an eyelash fortifying serum. An additional exemplary formulation for either a mascara, a serum, or an eyeliner is provided in Example 10 below.

The compositions will comprise effective amounts of creatine, N-biotinyl-gly-his-lys and other optional active(s), by which is meant an amount sufficient to impart to the formulated product one or more desired properties or modulatory activities, such as nourishing and enhancing the eyelashes.

The compositions can include, a cosmetically acceptable vehicle. A cosmetically acceptable vehicle refers to any vesicle, for a cosmetic, drug or medicament that is suitable for use in direct, safe contact with human tissues and/or human hair, including human eyelids and eyelashes, and may include, e.g., any diluent, solvent, carrier, filler, or the like. Such vehicles may take the form of any known in the art suitable for application to skin and/or hair, and may include, without limitation, hydrocarbons, glycerin, C₁₋₄ alcohols, fatty alcohols, fatty ethers, fatty esters, poyols, glycols, vegetable oils, mineral oils, liposomes, laminar lipid materials, silicone oils, and any combinations thereof.

The vehicle may comprise an aqueous phase, an oil phase, an alcohol phase, a silicone phase, individually or as mixtures thereof. The cosmetically acceptable vehicle may also comprise an emulsion. Non-limiting examples of suitable emulsions include water-in-oil emulsions, oil-in-water emulsions, silicone-in-water emulsions, water-in-silicone emulsions, wax-in-water emulsions, water-oil-water triple emulsions or the like, for example, having the appearance of a cream, gel or micro-emulsions. The emulsion may include an emulsifier, such as a nonionic, anionic or amphoteric surfactant.

The aqueous phase of the emulsion may include water, as well as one or more additional solvents, including lower alcohols, such as ethanol, isopropanol, and the like. The volatile solvent may also be a cosmetically acceptable ester such as butyl acetate or ethyl acetate; ketones such as acetone or ethyl ethyl ketone; or the like.

The oil phase of the emulsion preferably has one or more organic compounds, including emollients; humectants (such as butylene glycol, propylene glycol, Methyl gluceth-20, and glycerin); other water-dispersible or water-soluble components including thickeners such as veegum or hydroxyalkyl cellulose; gelling agents, such as high MW polyacrylic acid, i.e. CARBOPOL 934; and mixtures thereof. The emulsion may have one or more emulsifiers capable of emulsifying the various components present in the composition.

The oil phase may comprise one or more waxes, including for example, rice bran was, carnauba wax, ouricurry wax, candelilla wax, montan waxes, sugar cane waxes, ozokerite, polyethylene waxes, Fischer-Tropsch waxes, beeswax, botanical waxes, microcrystalline wax, silicone waxes, fluorinated waxes, and any combination thereof. The oil phase may comprise one or more volatile and/or non-volatile silicone oils.

Based on the teachings herein, a person skilled in the art will be able to select any of these vehicles, or any other materials described herein, and/or an amount thereof, and/or a proportion thereof, such that the lash nourishing and/or lash enhancing properties of the compositions of the instant invention can be conserved.

The composition may comprise one or more colorants. Suitable colorants, including pigments, lakes, and dyes, are well known in the art and are disclosed in the International Cosmetic Ingredient Dictionary and Handbook, 11th Edition, 2006 ("INCI"), the contents of which are hereby incorporated by reference. Organic pigments can include, for example, FD&C dyes, D&C dyes, including D&C Red, Nos. 2, 5, 6, 7, 10, 11, 12, 13, 30 and 34, D&C Yellow No. 5, Blue No. 1, Violet No. 2. Exemplary inorganic pigments include, but are not limited to, metal oxides and metal hydroxides such as magnesium oxide, magnesium hydroxide, calcium oxide, calcium hydroxides, aluminum oxide, aluminum hydroxide, iron oxides (α-Fe₂O₃, β- Fe₂O₃, Fe₃O₄, FeO), red iron oxide, yellow iron oxide, black iron oxide, iron hydroxides, titanium dioxide, titanium lower oxides, zirconium oxides, chromium oxides, chromium hydroxides, manganese oxides, cobalt oxides, cerium oxides, nickel oxides and zinc oxides and composite oxides and composite hydroxides such as iron titanate, cobalt titanate and cobalt aluminate. Other suitable colorants include ultramarine blue (i.e., sodium aluminum silicate containing sulfur), Prussian blue, manganese violet, bismuth oxychloride, talc, mica, sericite, magnesium carbonate, calcium carbonate, magnesium silicate, aluminum magnesium silicate, silica, titanated mica, iron oxide titanated mica, bismuth oxychloride, and the like. Further, one or more chroma-methicone colorants may be used, e.g., chroma-lite yellow-methocone, chroma-life red-methicone, and chroma-lite black-methicone. Whereas serum products are typically clear and colorless, mascara products are typically colored, and in particular black, brown, brown/black, and navy shades are contemplated for use with a mascara comprising a composition described herein.

The inventive compositions may optionally include preservatives. When present, the preservatives will include from about 0.01 to about 5% by weight, typically about 0.05 to about 4% by weight, and preferably about 0.1 to about 3% by weight % of the total composition.

In some preferred embodiments, the compositions of the invention comprise viscosity and/or pH modifiers. As noted above, addition on certain actives of the inventive compositions, such as creatine, N-biotinyl-gly-his-lys, and optional actives, to traditional Cosmetic formulations may have the effect of increasing pH and/or decreasing viscosity. Those of skill in the art will recognize that the pli and/or viscosity can be returned to suitable levels for cosmetic use by adding selected pH and/or viscosity modifiers.

The compositions of the invention may optionally comprise other active and inactive ingredients typically associated with cosmetic and personal care eye products, including, but not limited to, excipients, fillers, emulsifying agents, antioxidants, surfactants, additional film-formers, chelating agents, gelling agents, thickeners, emollients, humectants, moisturizers, vitamins, sodium ascorbyl/cholesteryl phosphate, minerals, viscosity and/or theology modifiers, sunscreens, keratolyics, retinoids, hormonal compounds, alpha-hydroxy acids, trioxaundecanedioic acid, alpha-keto acids, anti-mycobacterial agents, antifungal agents, antimicrobials, antivirus, analgesics, lipidic compounds, anti-allergenic agents, Hl or H2 antihistamine, ami-inflammatory agents, anti-irritants, antineoplastics, immune system boosting agents, immune system suppressing agents, anesthetics, antiseptics, skin cooling compounds, skin protectants, skin penetration enhancers, exfollients, lubricants, fragrances, additional colorants, staining agents, preservatives, stabilizers, pharmaceutical agents, photostabilizing agents, optical diffusers, UV absorbers, UV blockers, conditioners, and the like, and mixtures thereof. Based on the teachings herein, a person skilled in the art will be able to select any of these active or inactive ingredients, or any other materials described herein, and/or an amount thereof, and/or a proportion thereof, such that the desirable properties of the cosmetic compositions of the instant invention can be conserved.

In some embodiments, two or more different product formes may be provided together, e.g., as a kit. In certain particularly preferred embodiments, a kit is provided comprising a serum for topical application to eyelash follicles, and a mascara for topical application to eyelashes (e.g., including eyelash fibers and follicles), where the serum and mascara each comprise a composition according to the instant invention, in a cosmetically acceptable vehicle. In some embodiments, the kit further comprised a eyeliner comprising a composition described herein in a cosmetically acceptable vehicle. In some other embodiments, the eye liner may replace the serum or mascara product or the kit. Such kits will typically also include instructions for use, e.g., as taught herein. See example 3 below.

### EXAMPLES

### Example 1. Lash Transforming Mascara Formulation

An exemplary cosmetic composition comprising creatine and low level N-biotinyl-gly-his-lys for topical application to eyelashes is provided bellow. The composition is provided in the form of an emulsion mascara to be applied to the eyelashes for nourishing and volumizing the lashes. Amounts represent weight %.

| | |
|---|---|
| Biotinoyl tripeptide-1/oleanolic acid/PEG-40 Hydrogenated Castor Oil/PPG-26 Butcth-26/Butylene Glycol/Water¹ | 0.100 |
| Creatine | 0.500 |
| Protein/oligosaccharide blend-wheat² | 1.000 |
| Algae extract (Pelvetia canaliculata extract and Laminaria digitata)³ | 0.020 |
| Xymenynic acid | 0.010 |
| Tocopheryl acetate | 0.001 |
| Vehicle | qs |

| | |
|---|---|
| ¹ Biotinoyl tripeptide is present at 0.02 weight % active as supplied. ² Wheat protein is present al 23 weight % active as supplied. ³ Algae extract is present at 7 weight % active as supplied. The extract is with a propylene glycol-water mixture and preserved wilh ethyl and propyl parabens. | |

### Example 2. Lash Transforming Serum Formulation

Another exemplary cosmetic composition comprising creatine and low level N-biotinyl-gly-his-iys for topical application to eyelashes is provided below. The composition are provided in the form of an serum to be applied to the lash base for nourishing and volumizing the eyelashes. Amounts represent weight %.

| | |
|---|---|
| Biotinoyl tripeptide-l/oleanolic acid/PEG-40 Hydrogenated Castor Oil/PPG-26 Buteth-26/Butylene Glycol/Water (same active amount as in Example 1) | 0.100 |
| Creatine | 0.500 |
| Protein/oligosacch.blend-wheat (same active amount has in Example 1) | 1.000 |
| Algae extract (same active amount and mixture as in Example 1) | 0.020 |
| Xymenynic acid | 0.010 |
| Tocopheryl acetate | 0.001 |
| Vehicle | qs |

### Example 3. Clinical Efficiency Study

A clinical efficiency study was conducted to evaluate the effect of eye mascara and serum formulations according to Example 1 and 2, respectively. The study was a single center study, designed to determine the effect of the formulations on the volume, length, number and shine of eye lashes in a population of twenty-two (22) healthy adult female subjects, using digital image analysis techniques.

Twenty-two women of age 18 to 50 yrs competed this 12-week baseline-controlled mascara efficacy, study. In order to qualify for the study, subjects were required to have no visible signs or cuts, abrasions, excessive dryness, or erythema on or around the eyes and eyelids. Furthermore, each subject was offered her informed consent. Each subject who met the inclusion/exclusion criteria was told to refrain from using their eyelash products two days prior to the start of the study. Additionally, on the morning of their visits, subjects arrived without any eye make-up (e.g., mascara, eye shadow, eyeliner) on. The length and volume of eyelashes were determined before and after treatment with the serum and mascara products. The number and shine of the eyelashes were evaluated at base-line, and at week 4, 8 and 12 visits, without the product on. Images were captured for analysis as follows.

At the start of the study, before application of the test formulations, each subject groomed both her top and bottom eyelashes 14 times each with a lash brush provided by the testing facility. Following leash grooming, the subject was positioned in front of the camera and digital images of both eyes (fright and left, top and bottom lashes, with the subject looking up and looking straight at the camera) were captured and stored on an external hard drive. Additionally, photographs were taken with the subjects' eyes closed. All photos taken at this time point constituted baseline photos.

Baseline length and width for the top lashes were determined from the digital photographs using image analysis software (Image Pro, Media Cybernetics, Silver Spring, MD, USA). For the purposes of determining the baseline length and width of the top lashes the subject was instructed to look up and away from the camera. For the purposes of determining the baseline length and with for the bottom lashes, the subject was instructed to look straight at the camera. Additionally, the volume of the eye lashes (bottom and top) was calculated using the formula for a cone (the assumption being that the lash shape closely resembles that of a cone): Volume = 1/3 πr2L where: r = radius of the circle at the base of the cone (lash), such that r = ½ the width; and L = length of the cone. Finally, shine measurements were determined from an intensity histogram that was generated from photographs taken with the subjects' eyes closed. The intensity or the lashes, when the eyes are closed, were measured in gray levels (0 - 65,535). The average intensity value was recorded. The assumption was that shiner lashes should result in more light being reflected from the surface of the lash, producing higher gray levels. In addition to the above parameters, the total number of top and bottom lashes was counted.

After baseline photographs, the subject was provided with both the serum and mascara, products and instructed to apply them to both eyes as follows: (1) apply the eye serum at the base of the upper lashes; (2) after applying the serum, apply fourteen strokes of the mascara wand to the top lashes and fourteen to the bottom lashes, dipping the wand into the product between top and bottom lash application: and (3) approximately three minutes after applying the first coat, amply fourteen strokes of the mascara to top lashes in the same manner as the first coat (14 strokes of the mascara wand to the top lash and fourteen to the bottom lashes, dipping the wand into the product between top and bottom lash stroking). Therefore, the total number or applications of mascara to the top and bottom lashes was 28 strokes each.

Following product applications, additional photographs of the eyes where taken and these images constituted immediate photographs. The average length, width, and volume of the top and bottom eyelashes were deternined using digital imaging analysis on the baseline and immediate photographs. All photos were stored on an external hard drive.

Following immediate photographs, each subject was given the serum and mascara products and instructed to apply them at home in the evening in the same manner as applied earlier in the day and to continue applying the serum and mascara products daily for the next twelve weeks. Test subjects were given the following instructions: (1) apply the eye serum twice daily (morning and evening) at the base of the upper lashes; (2) in the morning, after applying the eye serum, apply the mascara to both the upper and lower lashes to provide a thick, heavy coverage and touch up the mascara in the afternoon. Further, a daily diary form was given to each subject to record the times of applications and to record any product performance and/or safety-related comments. Subjects were instructed to return to the testing facility in four, eight and twelve weeks for additional photographs. Subjects returning after four, eight and twelve weeks of using the serum and mascara products had photographs taken of their eyes in the same manner as at the baseline visit.

*Statistics*: The data were analyzed by an independent statistician using a truncation method. The significance of the mean charges was determined using a paired t-test. Statistical significance was set at p-values 0.05. All computations were performed using the Statistical Analysis System (SAS). The "up to" data were calculated at the 85^{th} percentile.

*Results*: Table 1 below shows the results of "instant" measurements of visible length and volume, following immediate use of the mascara formulation. In the Table, "A" represents the mean % improvement from baseline; "B" represents the "up to" % improvement from vaseline; while "C" represents the % of panelists showing improvements from the baseline readings.

**Table 1**

| | **Top Lashes** | | | **Bottom Lashes** | | | **Both Lashes** | | |
|---|---|---|---|---|---|---|---|---|---|
| | **A** | **B** | **C** | **A** | **B** | **C** | **A** | **B** | **C** |
| **Visible Length** | 42% | 86% | 100% | 37% | 64% | 96% | 39% | 51% | 96% |
| **Visible Volume** | 49% | 136% | 76% | 94% | 173% | 84% | 56% | 132% | 88% |

As Table 1 indicates, there was a significant increase in visible length and volume of the top and bottom lashes immediately after mascara application. These resets suggests that use of a mascara formulation comprising the inventive combination of creatine and low levels of N-biotinyl-gly-his-lys can produce instant improvements in visible length and volume of lashes.

Table 2 below shows the resuhs of "long-term" measurements following use of the serum plus mascara formulations. In the Table, "A" represents the mean % improvement from baseline; "B" represents the "up to" % improvement from baseline; while "C"' represents the % of panelists showing improvements from the baseline readings. "NS" means "non-significant"; while "NA" means "not applicable."

**Table 2**

| **Shine** | **Left Eve** | | | **Right Eye** | | | **Both Eves** | | |
|---|---|---|---|---|---|---|---|---|---|
| | **A** | **B** | **C** | **A** | **B** | **C** | **A** | **B** | **C** |
| **Week 4** | NS | NS | NS | NS | NS | NS | NS | NS | NS |
| **Week 8** | 23% | 47% | 90% | 18% | 38% | 85% | 20% | 40% | 95% |
| **Week 12** | 15% | 33% | 90% | 15% | 25% | 85% | 15% | 24% | 85% |
| | | | | | | | | | |

| **Visible Number** | **Top Lashes** | | | **Bottom Lashes** | | | **Both Lashes** | | |
|---|---|---|---|---|---|---|---|---|---|
| | **A** | **B** | **C** | **A** | **B** | **C** | **A** | **B** | **C** |
| **Week 4** | NS | NS | NS | NS | NS | NS | NA | NA | NA |
| **Week 8** | 27% | 69% | 73% | NS | NS | NS | NA | NA | NA |
| **Week 12** | 28% | 52% | 68% | NS | NS | NS | NA | NA | NA |

As Table 2 indicates, twice daily usage of the serum and mascara formulations, according to the instant disclosure, significantly improved the shine and the visible number of top leashes after 8 and 12 weeks. Notably, the lower lashes receiving only one application a day did not show a significant change in shine nor visible number, indicating a surprising criticality of dosage. These results suggests that more than one daily application of serum, mascara, or other formulations comprising the inventive combination or creatine and low levels of N-biotinyl-gly-his-lys can produce long-term improvements in lash shine and the number of visible lashes.

### Example 4. Increase in Keratin 5 production by Creatine

*In vitro* assays for keratin 5 indicate that creatine can increase keratin 5 production in keratinocytes at a concentration of 0.01 %.

The results suggest that compositions comprising creatine can be used to strengthen eyelashes, enhancing eyelash health and/or appearance, when topically applied to lash follicles.

### Example 5. increase in EMC production by N-biotinyl-gly-his-lys

*in vitro* assays indicate that N-biotinyl-gly-his-lys can increase production of collage IV, fibronectin and elastin extracellular matrix components in fibroblasts at concentrations of 2 x 10⁻⁵ % and 2 x 10⁻⁶ %.

The results suggest that compositions comprising the biotinylated peptide can be used to improve the extracellular matrix components of lash follicles, enhancing eyelash health and/or appearance, when topically applied thereto.

### Example 6. Inhibition of Mump-3 activity by N-biorinyl-gly-his-lys

*In vitro* assays indicate that N-biotinyl-gly-his-lys can decrease enzymatic activity of MP-3 in at a concentration of 2 x 10⁻⁷ %.

The results suggest that compositions comprising the biotinylated peptide can be used to protect extracellular matrix components of lash follicles, enhancing eyelash health and/or appearance, when topically applied thereto.

### Example 7. Inhibition of MMP-1/2 3, and 9) activity by algae extract

*In vitro* assays indicate that algae extract can inhibit enzymatic activity of MMP-1/2 3, and 9, at concentrations of 7 x 10⁻⁴ % and 7 x 10⁻⁵ %.

The results suggest that compositions comprising algae extract can be used to help protect the extracellular matrix components of lash follicles, enhancing eyelash health and/or appearance, when topically applied thereto.

### Example 8. Increase in HSP27 and 70 production by algae extract

*In vitro* assays using a reporter assay in embryonic kidney cells indicate that algae extract can increase production of HSPs 27 and 70, at a concentration of 0.007% and 1.4 x 10⁻³ %.

The results suggest that compositions comprising algae extract can be used to help protect the lashes, maintaining them in a healthy condition, when topically applied thereto.

### Example 9. Inhibition of Lipid Peroxidation by Tocopherol Acetate

*In vitro* assays indicate that tocopherol acetate can inhibit lipid peroxidation in keratinocytes, at a concentration of 0.001%.

The results suggest that compositions comprising tocopherol acetate can be used to help protect the lashes from oxidative damage, enhancing eyelash health and/or appearance, when topically applied thereto.

### Example 10. Lash Transforming Formulation

An exemplary cosmetic composition comprising creatine and low level N-biotinyl-gly-his-lys for topical application to eyelashes is provided below. The composition can be provided in the form of a mascara, cream, serum, eye liner to be applied to the eyelashes for nourishing and volumizing the lashes. Amounts represent weight %.

| | |
|---|---|
| Biotinoyl tripeptide-1/oleanolc acid/PEG-40 Hydrogenated Castor Oil/PPG-26 buteth-26/Butylene Glycol/Water (same active amount as in Example 1) | 0.100 |
| Creatine | 0.500 |
| vehicle | qs |

### Example 11. Lash Transforming Serum Formulation

Another exemplary cosmetic composition comprising creatine and low level N-biotinyl-gly-his-lys for topical application to eyelashes is provided below. The composition are provided in the form of an serum to be applied to the lash base for nourishing and volumizing the eyelashes. Amounts represent weight %.

| | |
|---|---|
| Biotinoyl tripeptide-1/oleanolic acid/PEG-40 Hydrogenated Castor Oil/PPG-26 Buteth-26/Butylene Glycol/Water (same active amount as in Example 1) | 1.0 |
| Creatine | 1.0 |
| Protein/olgosach.blend-wheat (same active amount as in Example I) | 2.0 |
| Algae extract (same active amount and mixture as in Example I) | 0.01 |
| Xymenynic acid | 0.02 |
| Tocopheryl acetate | 0.02 |
| vehicle | qs |

The terms used herein have their ordinary and accustomed meanings in the art, unless otherwise specified. All weights percentages referred to herein are given in terms of % by weight" or "weight %" of the total composition, which refers to the weight percent of the total formulation after addition of any carriers, solvents, emollient, or other components before application to eyelashes, unless otherwise indicated.

## Claims

1. A method for improving eyelash appearance comprising topically applying to eyelashes and/or eyelash follicles a composition comprising from 0.4 to 1.25 weight % creatine and from 1 x 10⁻⁶ to 3 x 10⁻⁴ weight % N-biotinyl-gly-his-lys in a cosmetically acceptable vehicle.

2. The method according to claim 1, wherein said composition is applied at least twice daily to said eyelash follicles,

3. The method according to claim, wherein said composition further comprises a wheat protein.

4. The method according to claim 3. wherein said composition further comprises at least one of a marine algae extract, tocopherol acetate, and at least 0.01 weight % xymenynic acid.

5. The method according to claim 1, wherein said composition does not comprise apigenin.

6. The method according to claim 1, wherein said improvement comprises at least one manifestation selected from the group consisting of:
(a) improvement in lash roof sheath thickness;
(b) improvement in lash anchorage;
(c) decrease in lash loss;
(d) reduction in lash breakage;
(e) increase in lash strength;
(f) improvement in lash growth rate;
(g) improvement in lash shine;
(h) improvement in the number of visible lashes;
(i) improvement in lash length; and
(j) improvement in lash volume.

7. The method according to claim 6, wherein said improvement composes an improvement in number of visible lashes.

8. The method according to claim 7, wherein said number of visible lashes increases by 50% after topical application of said composition to said eyelash follicles at least twice daily for twelve weeks.

9. The method according to claim 6, wherein said improvement comprises an improvement in lash volume and wherein said lash volume increases by 100% following topical application of said composition to said eyelashes.

10. A method for improving number of visible lashes on an eyelid, comprising:
applying to eyelash follicles of said eyelid a topical composition comprising from 0.4 to 1.25 weight % creatine and from 1 x 10⁻⁶ to 3 x 10⁻⁴ weight % N-biotinyl-gly-his-lys in a cosmetically acceptable vehicle at least twice daily; and
repeating said application until said number of visible lashes increases by 50%.

11. A topical composition comprising from 0.4 to 1.25 weight % creatine and from 1 x 10⁻⁶ to 3 x 10⁻⁴ weight % N-biotinyl-gly-his-lys in a cosmetically acceptable vehicle;
wherein said tropical composition is in the form of a serum, a mascara, or an eye liner.

12. The composition according to claim 11, wherein said N-biotinyl-gly-his-lys is present in an amount of no more than 2.5 x 10⁻⁴ weight %.

13. The composition according to any one of claims 11 or 12, further comprising a wheat protein.

14. The composition according to claim 13, further comprising at least one of a marine algae extract, tocopherol acetate, and at least 0.01 weight % xymenynic acid.

15. The composition according to any one of claims 11 or 12 wherein said composition does not comprise apigenin.

16. A kit comprising:
a serum for topical application to eyelash follicles, said senun comprising from 0.4 to 1.25 weight % creatine and from 1 x 10⁻⁶ x to 3 x 10⁻⁴ weight % N-biotinyl-gly-his-lys in a cosmetically acceptable vehicle; and
a mascara for topical application to eyelashes, said mascara comprising no more than 0.5 weight % creatine, and no more than 2.5 x 10⁻⁴ weight % N-biotinyl-gly-his-lys in a cosmetically acceptable vehicle.

17. A method for improving eyelash appearance comprising topically applying to eyelashes and/or eyelash follicles a composition comprising from 0.4 to 1.25 weight % and creatine from 1 x 10⁻⁶ to 3 x 10⁻⁴ N-biotinyl-gly-his-lys in a cosmetically acceptable vehicle, said composition being free of apigenin.

## Patentansprüche

1. Verfahren zur Verbesserung des Aussehens der Wimpern, umfassend die topische Anwendung einer Zusammensetzung, die 0,4 bis 1,25 Gew.-% Kreatin und 1 x 10⁻⁶ bis 3 x 10⁻⁴ Gew.-% N-Biotinyl-gly-his-lys in einem kosmetisch akzeptablen Träger umfasst, auf Wimpern und/oder Wimpernfollikel.

2. Verfahren nach Anspruch 1, wobei die Zusammensetzung mindestens zweimal täglich auf die Wimpernfollikel aufgebracht wird.

3. Verfahren nach Anspruch 1, wobei die Zusammensetzung ferner ein Weizenprotein umfasst.

4. Verfahren nach Anspruch 3, wobei die Zusammensetzung ferner zumindest einen der Bestandteile Meeresalgenextrakt, Tocopherolacetat und mindestens 0,01 Gew.-% Ximeninsäure umfasst.

5. Verfahren nach Anspruch 1, wobei die Zusammensetzung kein Apigenin umfasst.

6. Verfahren nach Anspruch 1, wobei die Verbesserung zumindest eine Erscheinungsform umfasst, die aus folgender Gruppe ausgewählt ist:
(a) Verbesserung der Dicke der Wimpernwurzelscheide;
(b) Verbesserung der Wimpernverankerung;
(c) Verringerung von Wimpernausfall;
(d) Verringerung von Wimpernbruch;
(e) Erhöhung der Wimpernfestigkeit;
(f) Verbesserung der Wimpernwachstumsrate;
(g) Verbesserung des Wimpernglanzes;
(h) Erhöhung der Anzahl sichtbarer Wimpern;
(i) Verbesserung der Wimpernlänge, und
(j) Verbesserung des Wimpernvolumens.

7. Verfahren nach Anspruch 6, wobei die Verbesserung eine Verbesserung der Anzahl sichtbarer Wimpern umfasst.

8. Verfahren nach Anspruch 7, wobei sich die Anzahl sichtbarer Wimpern nach mindestens zweimal täglicher topischer Anwendung der Zusammensetzung auf die Wimpernfollikel über zwölf Wochen um 50% erhöht.

9. Verfahren nach Anspruch 6, wobei die Verbesserung eine Verbesserung des Wimpernvolumens umfasst und sich das Wimpernvolumen nach topischer Anwendung der Zusammensetzung auf die Wimpern um 100% erhöht.

10. Verfahren zur Verbesserung der Anzahl sichtbarer Wimpern am Augenlid, umfassend:
mindestens zweimal tägliches Aufbringen einer topischen Zusammensetzung, die 0,4 bis 1,25 Gew.-% Kreatin und 1 x 10⁻⁶ bis 3 x 10⁻⁴ Gew.-% N-Biotinyl-gly-his-lys in einem kosmetisch akzeptablen Träger umfasst, auf die Wimpernfollikel des Augenlids, und
Wiederholen der Anwendung, bis sich die Anzahl sichtbarer Wimpern um 50% erhöht.

11. Topische Zusammensetzung, die 0,4 bis 1,25 Gew.-% Kreatin und 1 x 10⁻⁶ bis 3 x 10⁻⁴ Gew.-% N-Biotinyl-gly-his-lys in einem kosmetisch akzeptablen Träger umfasst,
wobei die topische Zusammensetzung in Form eines Serums, einer Wimperntusche oder eines Eyeliners vorliegt.

12. Zusammensetzung nach Anspruch 11, wobei das N-Biotinyl-gly-his-lys in einer Menge von nicht mehr als 2,5 x 10⁻⁴ Gew.-% vorliegt.

13. Zusammensetzung nach einem der Ansprüche 11 oder 12, ferner umfassend ein Weizenprotein.

14. Zusammensetzung nach Anspruch 13, ferner umfassend zumindest einen der Bestandteile Meeresalgenextrakt, Tocopherolacetat und mindestens 0,01 Gew.-% Ximeninsäure.

15. Zusammensetzung nach einem der Ansprüche 11 oder 12, wobei die Zusammensetzung kein Apigenin umfasst.

16. Kit, umfassend:
ein Serum zur topischen Anwendung auf Wimpernfollikel, wobei das Serum 0,4 bis 1,25 Gew.-% Kreatin und 1 x 10⁻⁶ bis 3 x 10⁻⁴ Gew.-% N-Biotinyl-gly-his-lys in einem kosmetisch akzeptablen Träger umfasst, und
eine Wimperntusche zur topischen Anwendung auf Wimpern, wobei die Wimperntusche nicht mehr als 0,5 Gew.-% Kreatin und nicht mehr als 2,5 x 10⁻⁴ Gew.-% N-Biotinyl-gly-his-lys in einem kosmetisch akzeptablen Träger umfasst.

17. Verfahren zur Verbesserung des Aussehens der Wimpern, umfassend die topische Anwendung einer Zusammensetzung, die 0,4 bis 1,25 Gew.-% Kreatin und 1 x 10⁻⁶ bis 3 x 10⁻⁴ Gew.-% N-Biotinyl-gly-his-lys in einem kosmetisch akzeptablen Träger umfasst, auf Wimpern und/oder Wimpernfollikel, wobei die Zusammensetzung frei von Apigenin ist.

## Revendications

1. Une méthode pour améliorer l'apparence du cil comprenant une application topique sur les cils et/ou les follicules des cils d'une composition comprenant de 0,4 à 1,25 en pourcentage en poids de créatine et de 1 x 10⁻⁶ à 3 x 10⁻⁴ en pourcentage en poids de N-biotinyl-gly-his-lys dans un vecteur cosmétiquement acceptable.

2. La méthode selon la revendication 1 où ladite composition est appliquée au moins deux fois par jour sur lesdits follicules des cils.

3. La méthode selon la revendication 1 où ladite composition comprend de plus une protéine de blé.

4. La méthode selon la revendication 3 où ladite composition comprend de plus au moins un extrait d'algue marine, de l'acétate de tocophérol et au moins 0,01 en pourcentage en poids d'acide xyménynique.

5. La méthode selon la revendication 1 où ladite composition ne comprend pas d'apigénine.

6. La méthode selon la revendication 1 où ladite amélioration comprend au moins une manifestation sélectionnée à partir du groupe se composant de :
(a) amélioration de l'épaisseur de la gaine de la racine des cils ;
(b) amélioration de la fixation des cils ;
(c) diminution de la perte de cils ;
(d) réduction de la cassure des cils ;
(e) augmentation de la force des cils ;
(f) amélioration du taux de croissance des cils ;
(g) amélioration de la brillance des cils ;
(h) amélioration du nombre de cils visibles ;
(i) amélioration de la longueur des cils ; et
(j) amélioration du volume des cils.

7. La méthode selon la revendication 6 où ladite amélioration comprend une amélioration du nombre de cils visibles.

8. La méthode selon la revendication 7 où ledit nombre de cils visibles augmente de 50% après l'application topique de ladite composition sur les follicules des cils au moins deux fois par jour pendant 12 semaines.

9. La méthode selon la revendication 6 où ladite amélioration comprend une amélioration du volume des cils et où ledit volume des cils augmente de 100% après l'application topique de ladite composition sur les cils.

10. Une méthode pour améliorer le nombre de cils visibles sur une paupière, comprenant :
L'application sur les follicules des cils de ladite paupière d'une composition topique comprenant de 0,4 à 1,25 en pourcentage de poids de créatine et de 1 x 10⁻⁶ à 3 x 10⁻⁴ en pourcentage en poids de N-biotinyl-gly-his-lys dans un vecteur cosmétiquement acceptable au moins deux fois par jour ; et
La répétition de ladite application jusqu'à ce que le nombre de cils visibles augmente de 50%.

11. Une composition topique comprenant de 0,4 à 1,25 en pourcentage de poids et de 1 x 10⁻⁶ à 3 x 10⁻⁴ en pourcentage de poids de N-biotinyl-gly-his-lys dans un vecteur cosmétiquement acceptable ;
Où ladite composition topique est sous forme de sérum, de mascara, ou d'eye liner.

12. La composition selon la revendication 11, où le N-biotinyl-gly-his-lys est présent à hauteur de 2,5 x 10⁻⁴ en pourcentage de poids maximum.

13. La composition selon l'une des revendications 11 ou 12, comprenant de plus une protéine de blé.

14. La composition selon la revendication 13, comprenant de plus au moins un extrait d'algue marine, de l'acétate de tocophérol, et au moins 0,01 en pourcentage de poids d'acide xyménynique.

15. La composition selon l'une des revendications 11 ou 12, où ladite composition ne comprend pas d'apigénine.

16. Un kit comprenant :
Un sérum pour l'application topique sur les follicules des cils, ledit sérum comprenant de 0,4 à 1,25 en pourcentage de poids de créatine et de 1 x 10⁻⁶ à 3 x 10⁻⁴ en pourcentage en poids de N-biotinyl-gly-his-lys dans un vecteur cosmétiquement acceptable ; et
Un mascara pour une application topique sur les cils, ledit mascara comprenant maximum 0,5 en pourcentage de poids et 2,5 x 10⁻⁴ maximum en pourcentage en poids de N-biotinyl-gly-his-lys dans un vecteur cosmétiquement acceptable.

17. Une méthode pour améliorer l'apparence des cils comprenant une application topique sur les cils et/ou les follicules des cils d'une composition comprenant de 0,47 à 1,25 en pourcentage en poids de créatine et de 1 x 10⁻⁶ à 3 x 10⁻⁴ en pourcentage en poids de N-biotinyl-gly-his-lys dans un vecteur cosmétiquement acceptable, ladite composition ne contenant pas d'apigénine.
